# FASCICULE DE BREVET EUROPEEN

(11) **EP 0 748 632 B1**
(45) Date de publication et mention de la délivrance du brevet: **20.03.2002**
(21) Numéro de dépôt: 96390005.5
(22) Date de dépôt: 03.06.1996
(51) Int. Cl.: A61L 2/00, A61L 2/16

(54) **Procédé de traitement antiviral des tissus biologiques à trame collagénique**
Verfahren zur antiviralen Behandlung von biologischen Geweben mit Kollagentextur
Method for anti-viral treatment of biological tissues with collagenic texture

(30) Priorité: 13.06.1995 FR 9507266
(43) Date de publication de la demande: 18.12.1996
(73) Titulaire: BIOLAND Société à Responsabilité Limitée, F-31100 Toulouse (FR)
(72) Inventeur: Fages, Jacques, 31120 Portet sur Garonne (FR); Frayssinet, Patrick, 31710 Saiguede (FR); Bonel, Gilbert, 31100 Toulouse (FR)
(74) Mandataire: Cabinet BARRE LAFORGUE & associés

(56) Documents cités:
- EP-A- 0 603 920
- WO-A-93/17724
- WO-A-94/03590

## Description

L'invention concerne un procédé de traitement, dans un but de prophylaxie antivirale, des tissus biologiques à trame collagénique, tels que les tissus osseux, tendineux, cartilagineux, ou ligamenteux d'origine humaine ou animale, pour l'obtention d'un biomatériau implantable dans le corps humain ou animal.

Les tissus biologiques à trame collagénique tels que les tissus osseux peuvent, après traitement, servir de greffons (allogreffe ou xénogreffe). En particulier, un tel tissu peut servir de biomatériau entrant dans la composition d'une prothèse implantable de tissu de soutien (os, cartilage, tendon, ligament), c'est-à-dire incorporé dans une telle prothèse ou constituant une telle prothèse.

Avant l'implantation, le tissu doit subir des traitements de préparation et de purification.

Ainsi, EP-A-0.603.920 décrit un procédé de traitement de tissus osseux dans lequel on fait pénétrer un fluide à l'état supercritique dans la totalité du tissu osseux, ce qui a pour effet d'en extraire les matières organiques essentiellement lipidiques et d'en renforcer la résistance mécanique. D'autres étapes du procédé sont prévues pour l'obtention du biomatériau implantable, à savoir des étapes préalables de nettoyage mécanique et découpe, et des étapes subséquentes de traitement chimique (par le peroxyde d'hydrogène) et/ou enzymatique (protéase) pour en extraire les protéines résiduelles, lavage, déshydratation et désinfection dans des bains d'éthanol (cette dernière étape permettant d'accroître la sécurité infectieuse du biomatériau compte tenu de ses propriétés virucides).

En outre, les procédés utilisés jusqu'à maintenant pour limiter les risques de contamination virale des tissus osseux (tels que les os provenant des banques d'os) consistent soit à soumettre le tissu à un rayonnement gamma ou beta, soit à le stériliser à l'oxyde d'éthylène ou par traitement thermique.

L'irradiation aux rayons gamma ou beta ne permet pas de détruire avec certitude tous les virus. En effet, certains virus sont radiorésistants. En outre, cette irradiation diminue, selon la dose de rayons appliquée, la résistance mécanique de l'os.

La stérilisation à l'oxyde d'éthylène pose le problème de la toxicité de ce composé chimique, et du produit qu'il forme en présence d'eau (éthylène glycol). En outre, l'oxyde d'éthylène modifie la structure osseuse et affecte les propriétés d'ostéoinduction.

La stérilisation à la chaleur détruit les propriétés mécaniques du collagène et donc des tissus osseux.

Par ailleurs, la destruction mécanique de cellules microbiennes par une décompression brutale dans le dioxyde de carbone CO₂ à l'état supercritique a déjà été décrite ("Disruption of Microbial Cells by the Flash Discharge of High-Pressure Carbon Dioxyde", Kozo Nakamura et al., Biosci. Biotech. Biochem., 58(7), 1297-1301, 1994).

Néanmoins, aucune technique n'assure une totale décontamination des tissus sans affecter les propriétés mécaniques et de biocompatibilité du tissu.

L'invention vise donc à proposer un nouveau procédé de traitement prophylactique antiviral des tissus à trame collagénique, et plus particulièrement des tissus osseux, d'origine humaine ou animale, pour l'obtention de biomatériaux implantables exempts de toute contamination virale et aptes à être utilisés comme prothèses de tissu de soutien ou dans des prothèses de tissu de soutien.

L'invention vise aussi à proposer un tel traitement qui, simultanément, préserve les caractéristiques biomécaniques et de biocompatibilité du tissu, voire les renforce.

En particulier, l'invention vise à proposer un procédé de traitement antiviral d'un tissu osseux (tel qu'un tissu osseux provenant d'une banque d'os), avant son implantation dans le corps humain ou animal lors d'une greffe (allogreffe ou xénogreffe) osseuse, procédé qui assure l'élimination de toute contamination virale éventuelle et permet ainsi l'utilisation de ces tissus osseux sans risque.

L'invention concerne donc un procédé selon la revendication 1.

Un procédé selon l'invention est applicable pour le traitement de tout tissu à trame collagénique apte à supporter les étapes du traitement et notamment des conditions de pression et de température où le dioxyde de carbone est à l'état supercritique. Le tissu à trame collagénique peut être en phase solide, divisé (en poudre) ou non divisé (en morceaux ou blocs), et poreux.

Le procédé selon l'invention permet donc de lutter contre les virus et procure un traitement préventif antiviral des tissus à trame collagénique d'origine humaine ou animale tels que les tissus osseux.

Aucune explication théorique ne peut être donnée avec certitude à l'effet antiviral ou virucide du procédé selon l'invention, et notamment du dioxyde de carbone à l'état supercritique dans les tissus à trame collagénique. Néanmoins, il est probable que la molécule du dioxyde de carbone, de nature organique, présente aux conditions supercritiques, d'une part la faculté de diffuser à travers la totalité de la matière poreuse du tissu à trame collagénique, et d'autre part une réactivité biochimique forte avec les molécules organiques des composés biochimiques nécessaires au développement et à l'activité des virus (enzymes, constituants protéiniques, capsides, acides nucléiques, enveloppes...).

Avantageusement, l'invention concerne aussi un procédé dans lequel on utilise un courant de dioxyde de carbone CO₂ à l'état supercritique au contact du tissu à trame collagénique, à raison de 100 à 500 -notamment de l'ordre de 300-grammes de dioxyde de carbone supercritique par gramme de tissu à trame collagénique à traiter.

Avantageusement, selon l'invention, on laisse ledit courant de dioxyde de carbone CO₂ à l'état supercritique au contact d'un tissu à trame collagénique pendant une durée déterminée en fonction du débit dudit courant pour permettre le passage de 100 à 500 -notamment de l'ordre de 300- grammes de dioxyde de carbone supercritique par gramme de tissu.

Plus particulièrement, avantageusement et selon l'invention, le dioxyde de carbone supercritique est placé à une pression comprise entre 1.10⁷ et 5.10⁷ Pa -notamment de l'ordre de 2,5.10⁷ Pa- et à une température comprise entre 40° C et 55° C, -notamment de l'ordre de 50° C-.

En outre, l'invention concerne aussi un procédé comprenant avantageusement une ou plusieurs des caractéristiques suivantes :
- on met en contact le tissu à trame collagénique avec une solution de peroxyde d'hydrogène H₂O₂ dont le titre massique est supérieur à 1 %, notamment de l'ordre de 35 % ; et à une température comprise entre 30° C et 50° C, notamment de l'ordre de 40° C ;
- on met en contact le tissu à trame collagénique avec une solution d'hydroxyde de sodium NaOH dont le titre massique est compris entre 3 % et 15 % -notamment de l'ordre de 4 %- ; puis on effectue un ou plusieurs rinçage(s) à l'eau et une neutralisation par passage dans une solution neutralisante, par exemple une solution de NaH₂PO₄ ;
- on met en contact le tissu à trame collagénique successivement avec une solution d'éthanol dont le titre en volume est supérieur à 90 %, notamment de l'ordre de 95 %, puis avec une solution d'éthanol dont le titre en volume est supérieur à 95 %, notamment de l'ordre de 100 %.

Avantageusement et selon l'invention, pour effectuer la mise en contact du tissu à trame collagénique avec chacune des solutions sus-mentionnées, on plonge le tissu dans ladite solution.

La succession des étapes du procédé selon l'invention (traitement par le dioxyde de carbone CO₂ à l'état supercritique, puis traitement au peroxyde d'hydrogène H₂O₂, puis traitement à l'hydroxyde de sodium NaOH, puis traitement à l'éthanol) garantit seule l'élimination de toute présence virale et de toute forme d'activité virale dans le tissu traité et présente un effet cumulatif et synergique inattendu vis-à-vis de toutes sortes de virus. En outre, l'intégrité biomécanique est préservée.

L'invention est plus particulièrement applicable pour lutter contre les virus de la famille des Togaviridae, notamment du genre Alphavirus, tels que le virus de l'Hépatite C, et prévenir leur transmission lors des greffes de tissus ; pour lutter contre les virus de la famille des Picormaviridae, notamment du genre Enterovirus, plus particulièrement le virus Polio Sabin, et prévenir leur transmission lors des greffes de tissus ; pour lutter contre les virus de la famille des Herpesviridae et prévenir leur transmission lors des greffes de tissus ; pour lutter contre les virus de la famille des Retroviridae, notamment du genre Lentivirus, plus particulièrement les virus de l'Immunodéficience humaine VIH, et prévenir leur transmission lors des greffes de tissus.

Grâce à l'invention, on peut pour la première fois, envisager une exploitation sûre et industrielle des tissus en provenance de banques.

Les exemples ci-après illustrent l'invention.

### PROTOCOLE EXPERIMENTAL :

Dans les exemples, on teste la capacité de chaque étape du traitement à éliminer et/ou inactiver les virus : 1) en contaminant les échantillons avec une quantité significative de virus, puis 2) en effectuant l'étape de traitement considérée sur les échantillons, puis 3) en déterminant la quantité de virus éliminée et/ou inactivée durant cette étape.

Les échantillons testés sont des quarts de tête de fémur d'origine humaine.

Les expérimentations sont conduites par l'Institut Pasteur (PARIS, FRANCE) selon le protocole n° 5151-A1, et conformément aux réglementations communautaires françaises, américaines et de l'O.C.D.E., des Bonnes Pratiques de Laboratoire.

Pour chaque expérimentation (c'est-à-dire pour chaque étape testée et chaque virus), on réalise des contrôles positifs (stock de virus de même concentration que celle utilisée pour la contamination de l'échantillon, immédiatement congelé à -75° C ; stock de virus de même concentration, ultracentrifugé, aliquoté et congelé à -75° C ; stock de virus de même concentration, laissé à température ambiante pendant le temps de l'expérience, puis ultracentrifugé, aliquoté et congelé à -75° C ; échantillon contaminé dans les mêmes conditions et dont l'activité virale est immédiatement testée ; échantillon contaminé dans les mêmes conditions, non soumis à l'étape de traitement mais laissé sous hotte à température ambiante pendant la durée de l'étape de traitement, puis dont l'activité virale est testée) et des contrôles négatifs (milieu de culture utilisé pour les titrages ; échantillon mis en présence de milieu de culture, puis soumis à l'étape de traitement, puis dont l'activité virale est testée).

L'activité virale d'un échantillon osseux est testée par un protocole dont la fiabilité est préalablement vérifiée sur un échantillon de même nature que ceux utilisés dans l'étape de traitement à tester, et contaminé avec un virus de résistance moyenne aux traitements physico-chimiques (virus de la pseudorage porcine).

Ce protocole est le suivant. Les quarts de tête de fémur sont morcelés et incubés dans le milieu de culture pendant une heure à 4° C. Un premier volume V1 de surnageant est récupéré. Les morceaux sont ensuite centrifugés à 400 g pendant 2 min, et on récupère un deuxième volume V2 de surnageant. Le mélange des deux volumes V1+V2 de surnageant est centrifugé à 1 550 g pendant 10 min pour éliminer les débris osseux, et le surnageant est ultracentrifugé, aliquoté et congelé à - 75° C. L'activité virale est titrée selon une procédure normalisée pour chaque virus, par dosage de l'activité transcriptase inverse (pour le virus VIH-1), ou par dénombrement des plages de lyse sur tapis cellulaire monocouche (pour les virus Sindbis, Polio Sabin 1, pseudorage porcine). Pour le titrage, on utilise des parties aliquotées décongelées extemporanément à 37° C et placées dans de la glace fondante. Un contrôle de cytotoxicité est effectué à chaque expérimentation. La limite de détection est déterminée par le plus faible titre théorique obtenu, ou selon la formule de Poisson si aucune production virale n'est observée. Tous les essais sont réalisés en double.

Pour tester l'effet antiviral de l'étape n° 1 de traitement par un courant de dioxyde de carbone à l'état supercritique, on procède de la façon suivante.

On réalise des perçages de 4 mm de diamètre et de 1 à 1,5 cm de profondeur dans les blocs osseux que l'on lave dans deux bains successifs de 100 ml de milieu de culture à 1 %(v/v) de sérum de veau foetal pendant 10 min sous agitation douce. On sèche les blocs osseux en étuve pendant 2 ou 3 jours, puis on teste la capacité des perçages à retenir les liquides en y introduisant du milieu de culture. Si une fuite est observée après 1 à 2 min, soit le perçage est colmaté avec du ciment biologique (dont l'absence d'effet inactivant sur les virus a été préalablement vérifiée), soit l'échantillon est éliminé. Les échantillons retenus sont séchés pendant une durée complémentaire de 2 à 3 jours.

Chaque échantillon est ensuite contaminé par introduction d'une suspension virale dans les perçages qui sont ensuite rebouchés avec le ciment biologique.

Les échantillons contaminés sont ensuite traités par un courant de dioxyde de carbone à l'état supercritique pendant une durée de 10 min par gramme de l'échantillon. Pour ce faire on utilise le procédé et le dispositif décrit dans EP-A-0.603.920.

L'étape n° 2 du traitement est un passage dans un bain de solution à 35 % (m/m) de peroxyde d'hydrogène H₂O₂ à 40° C dont la durée est déterminée par un test de flottaison (jusqu'à ce que l'échantillon de contrôle négatif coule).

Pour tester l'effet antiviral de l'étape n° 2, on utilise des quarts de tête de fémur ayant été préalablement traités au dioxyde de carbone supercritique (10 min par gramme d'échantillon) comme indiqué ci-dessus. Les échantillons sont lavés dans deux bains successifs de milieu de culture puis séchés 24 h avant d'être contaminés en ajoutant 1 ml de la suspension virale (ou du milieu de culture pour l'échantillon de contrôle négatif) goutte à goutte sur chacune des faces de l'échantillon. La pénétration de la suspension virale dans un échantillon est préalablement vérifiée avec un colorant.

L'activité virale est mesurée avant et après le traitement au peroxyde d'hydrogène comme indiqué ci-dessus.

L'étape n° 3 du traitement est un passage dans un bain de solution de NaOH 1M à 20° C pendant 1 h, suivi d'un rinçage à l'eau et d'une neutralisation par passage dans un bain de NaH₂PO₄ (12 g/l) pendant 10 min. Pour tester l'effet antiviral de l'étape n° 3, on utilise des quarts de tête de fémur ayant été préalablement traités successivement par le CO₂ supercritique, puis par le peroxyde d'hydrogène H₂O₂ comme indiqué ci-dessus. Les échantillons sont ensuite contaminés comme indiqué ci-dessus pour l'étape n° 2. L'activité virale est mesurée avant et après le traitement à l'hydroxyde de sodium comme indiqué ci-dessus.

L'étape n° 4 du traitement consiste en un passage dans un bain d'éthanol à 95 % (v/v) pendant 3 h à température ambiante, suivi d'un passage dans un bain d'éthanol à 100 % (v/v) pendant 2 h à température ambiante.

Pour tester l'effet antiviral de l'étape n° 4, on utilise des quarts de tête de fémur ayant été préalablement traités successivement par le CO₂ supercritique, le peroxyde d'hydrogène et l'hydroxyde de sodium comme indiqué ci-dessus. Les échantillons sont ensuite contaminés comme indiqué pour l'étape n° 2 et l'activité virale est mesurée avant et après traitement par l'étape n° 4 comme indiqué ci-dessus.

A partir des résultats expérimentaux, on calcule, pour chaque traitement, le facteur de réduction représenté par le logarithme en base 10 du rapport entre la quantité totale de virus contaminant l'échantillon avant le traitement et la quantité totale de virus contaminant l'échantillon après le traitement. Le facteur de réduction du traitement est la somme du logarithme en base 10 du rapport de la charge virale totale dans le milieu de culture sur la charge virale totale dans l'échantillon avant le traitement (si ce logarithme est supérieur à 1), et du logarithme en base 10 du rapport de la charge virale totale dans l'échantillon avant le traitement sur la charge virale totale dans l'échantillon après l'étape de traitement.

Un traitement est inactivant sur un virus à partir d'un facteur de réduction de 4, et totalement inactivant si le facteur de réduction est supérieur à 6.

A noter que l'effet antiviral de chaque étape étant testé à partir d'os ayant subi successivement les étapes précédentes, les facteurs de réduction obtenus sont cumulatifs et la somme de ces facteurs de réduction pour les quatre étapes représente l'effet antiviral du procédé complet.

Chaque essai est considéré comme valide lorsque le titre viral attendu est obtenu avec le contrôle positif (stock de virus de même concentration immédiatement congelé), et si aucune plage ou aucune production virale n'est obtenue avec les contrôles négatifs.

### EXEMPLE 1

L'effet du traitement antiviral selon l'invention est testé sur le virus du SIDA HIV-1, qui est un rétrovirus à ARN enveloppé de la famille des Retrovividae, sous-famille des Lentivirinae, genre Lentivirus, sphérique de 80 à 130 nm, dont le génome est constitué de deux molécules d'ARN simple brin identiques de 9,2 kb. Ce virus est faiblement résistant aux traitements physico-chimiques. Il est obtenu à partir de surnageants de culture de cellules humaines infectées.

Les résultats obtenus sont donnés dans le tableau 1 suivant :

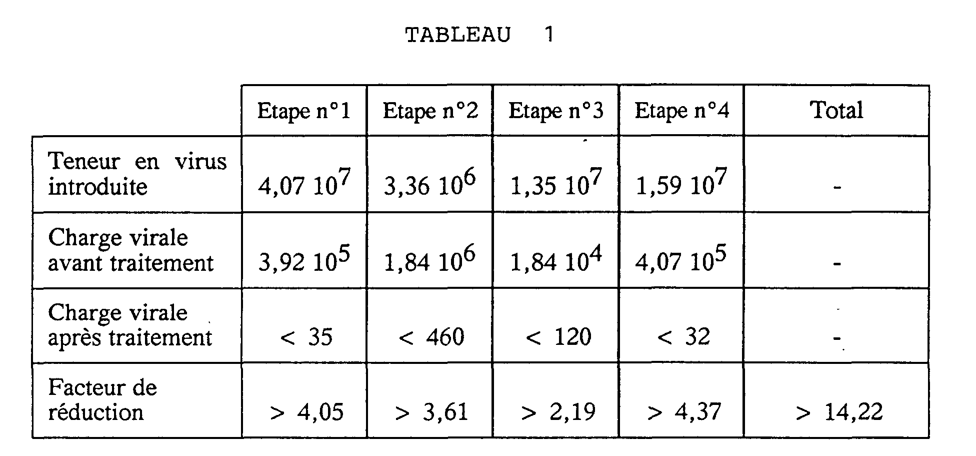

Dans tous les cas la limite de détection après traitement n'est pas atteinte, aucun virus n'ayant pu être récupéré. En conséquence les facteurs de réduction sont des valeurs minimales. Les vraies valeurs peuvent être largement supérieures. Bien qu'aucun virus n'ait été détecté après traitement, les facteurs de réduction obtenus aux étapes n° 2 et 3 sont relativement faibles du fait soit d'une limite de détection élevée, soit de la faible charge virale avant traitement liée à un effet inactivant de l'os lui-même.

Comme on le voit, le procédé complet (étapes 1 à 4) a un facteur de réduction supérieur à 14,22, et est donc totalement inactivant vis-à-vis du virus.

### EXEMPLE 2

L'effet du traitement antiviral de l'invention est testé sur le virus Sindbis, qui est un togavirus à ARN enveloppé. Ce virus est considéré comme un modèle pour celui de l'Hépatite C qui est, après le SIDA, la deuxième maladie infectieuse la plus menaçante pour la santé publique dans le cadre des risques liés aux greffes de tissus. Ce virus appartient à la famille des Togaviridae, genre Alphavirus. C'est un virus enveloppé, sphérique de 60 à 70 nm de diamètre. Son génome est constitué d'une molécule d'ARN simple brin de 12 kb. Il est faiblement résistant aux traitements physico-chimiques. Il est obtenu à partir de surnageants de culture de cellules rénales de singe infectées.

Le tableau 2 suivant donne les résultats obtenus.

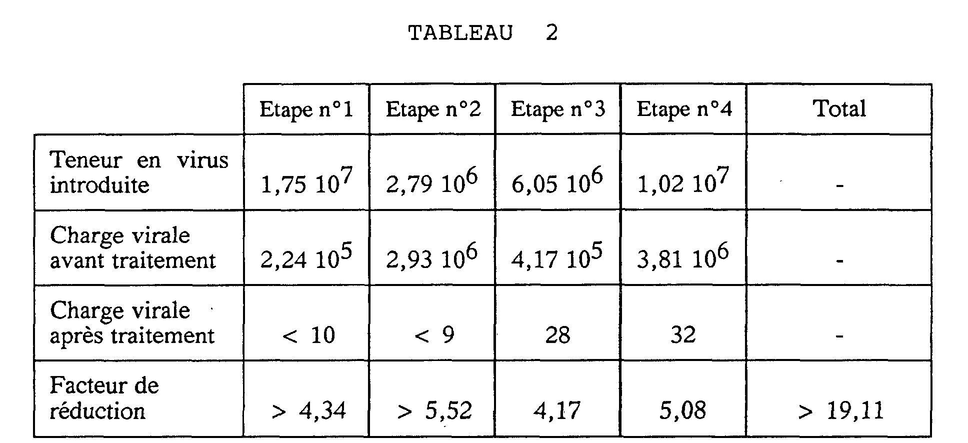

Dans les étapes n° 1 et n° 2, la limite de détection après traitement n'est pas atteinte, aucun virus n'ayant pu être récupéré. En conséquence les facteurs de réduction sont des valeurs minimales. Les vraies valeurs peuvent être largement supérieures. On constate aussi que le procédé complet (étapes 1 à 4) a un facteur de réduction supérieur à 19,11, et est donc totalement inactivant vis-à-vis du virus.

### EXEMPLE 3

L'effet du traitement antiviral de l'invention a été testé sur le virus Polio Sabin 1, qui est un picornavirus à ARN non-enveloppé. Comme tous les virus non-enveloppés, il est plus résistant vis-à-vis des traitements physico-chimiques que les virus enveloppés. Ce virus appartient à la famille des Picornaviridae, genre Enterovirus. C'est un virus non-enveloppé, de symétrie icosaédrique de 28 à 30 nm de diamètre. Son génome est constitué d'une molécule d'ARN simple brin de polarité positive de 7,4 kb. Sa résistance aux traitements physico-chimiques est moyenne à forte. Il est obtenu à partir de surnageants de culture de cellules humaines infectées.

Le tableau 3 suivant donne les résultats obtenus.

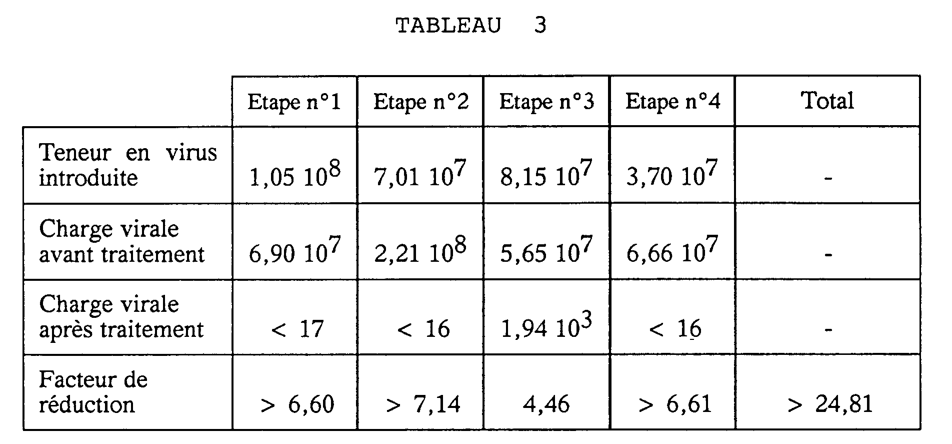

Dans les cas où le chiffre de la charge virale après traitement est précédé du signe <, la limite de détection après traitement n'est pas atteinte, aucun virus n'ayant pu être récupéré. En conséquence, les facteurs de réduction correspondants sont des valeurs minimales. Les vrais valeurs peuvent être largement supérieures.

On constate encore que le procédé complet (étapes 1 à 4) a un facteur de réduction supérieur à 24,81, et est donc totalement inactivant vis-à-vis du virus.

### EXEMPLE 4

L'effet du traitement antiviral de l'invention est testé sur le virus de la pseudorage porcine, qui est un herpèsvirus à ADN enveloppé. Ce virus appartient à la famille des Herpesviridae, sous-famille des alphaherpesvirinae, genre Varicellavirus. C'est un virus enveloppé, de symétrie icosaédrique dont la taille varie de 100 à 200 nm. Son génome est constitué d'une molécule d'ADN double brin linéaire de 150 à 200 kb. Il a une résistance moyenne aux traitements physico-chimiques. Il est obtenu à partir de surnageants de culture de fibroblastes d'embryons de lapin infectés.

Le tableau 4 suivant donne les résultats obtenus.

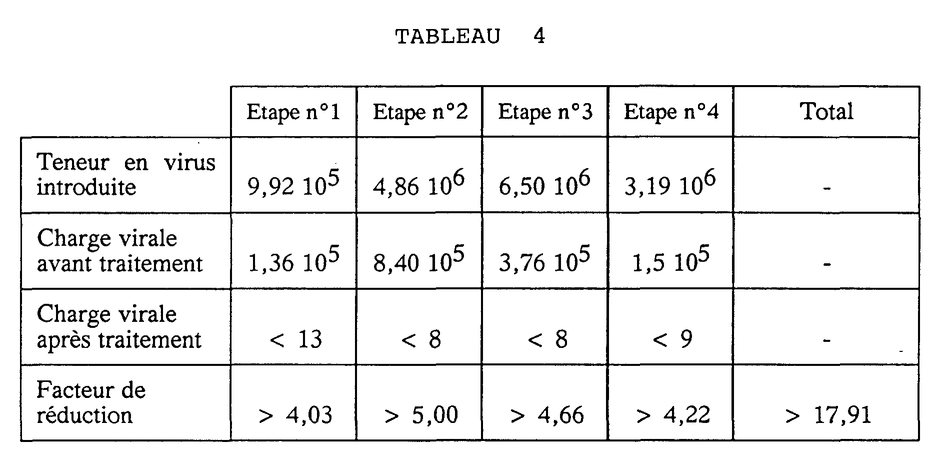

Dans tous les cas la limite de détection après traitement n'est pas atteinte, aucun virus n'ayant pu être récupéré. En conséquence, les facteurs de réduction sont des valeurs minimales. Les vraies valeurs peuvent être largement supérieures. Là encore, le procédé complet a un facteur de réduction (supérieur à 17,91) qui assure l'élimination de toute contamination virale.

Les exemples 1 à 4 ci-dessus démontrent l'effet antiviral du traitement par les étapes n° 1 à 4 successives sur des virus de référence variés (virus à ARN ou à ADN, enveloppés ou non, résistants ou non aux traitements physico-chimiques...), dans des conditions normalisées et donc l'efficacité de l'invention à titre d'antiviral. En effet, les virus testés sont choisis en conformité avec la réglementation européenne sur les validations virales (Guideline III/8115/89) et sur les dérivés du sang (Guideline III/83179/89).

## Revendications

1. Procédé de traitement, dans un but de prophylaxie antivirale, d'un tissu à trame collagénique destiné à servir de biomatériau, **caractérisé en ce qu'**on effectue successivement:
- une étape de traitement par le dioxyde de carbone CO₂ à l'état supercritique, au cours de laquelle on place le tissu à trame collagénique au contact d'un courant de dioxyde de carbone CO₂ à l'état supercritique qui est adapté pour présenter une fonction d'élimination de la contamination virale au sein du tissu,
- une étape de traitement au peroxyde d'hydrogène H₂O₂, au cours de laquelle on met le tissu à trame collagénique en contact avec une solution de peroxyde d'hydrogène H₂O₂ adaptée pour présenter une fonction d'élimination de la contamination virale du tissu,
- une étape de traitement à l'hydroxyde de sodium NaOH, au cours de laquelle on met le tissu à trame collagénique en contact avec une solution d'hydroxyde de sodium NaOH adaptée pour présenter une fonction d'élimination de la contamination virale du tissu,
- une étape de traitement à l'éthanol, au cours de laquelle on met le tissu à trame collagénique en contact avec une solution d'éthanol CH₃CH₂OH adaptée pour présenter une fonction d'élimination de la contamination virale du tissu, ces étapes étant adaptées pour que leur succession assure l'élimination de toute contamination virale dans le tissu traité.

2. Procédé selon la revendication 1, **caractérisé en ce qu'**on utilise un courant de dioxyde de carbone CO₂ à l'état supercritique au contact du tissu à trame collagénique, à raison de 100 à 500 grammes de dioxyde de carbone supercritique par gramme de tissu.

3. Procédé selon l'une des revendications 1 et 2, **caractérisé en ce qu'**on laisse ledit courant de dioxyde de carbone CO₂ à l'état supercritique au contact du tissu à trame collagénique pendant une durée déterminée en fonction du débit dudit courant pour permettre le passage de 100 à 500 grammes de dioxyde de carbone supercritique par gramme de tissu.

4. Procédé selon l'une des revendications 1 à 3, **caractérisé en ce que** le dioxyde de carbone CO₂ supercritique est placé à une pression comprise entre 1.10⁷ et 5.10⁷ Pa et à une température comprise entre 40° C et 55° C.

5. Procédé selon l'une des revendications 1 à 4, **caractérisé en ce qu'**on met le tissu à trame collagénique en contact avec une solution de peroxyde d'hydrogène H₂O₂ dont le titre massique est supérieur à 1 %.

6. Procédé selon l'une des revendications 1 à 5, **caractérisé en ce qu'**on met le tissu à trame collagénique en contact avec une solution de peroxyde d'hydrogène H₂O₂ dont le titre massique est de l'ordre de 35 %.

7. Procédé selon l'une des revendications 1 à 6, **caractérisé en ce qu'**on met le tissu à trame collagénique en contact avec une solution de peroxyde d'hydrogène à une température comprise entre 30° C et 50° C.

8. Procédé selon l'une des revendications 1 à 7, **caractérisé en ce qu'**on met le tissu à trame collagénique en contact avec une solution d'hydroxyde de sodium NaOH dont le titre massique est compris entre 3 % et 15 %.

9. Procédé selon l'une des revendications 1 à 7, **caractérisé en ce qu'**on met le tissu à trame collagénique en contact avec une solution d'hydroxyde de sodium NaOH dont le titre massique est de l'ordre de 4 %.

10. Procédé selon l'une des revendications 1 à 9, **caractérisé en ce que** lors de l'étape de traitement à l'hydroxyde de sodium, on met le tissu à trame collagénique en contact avec une solution d'hydroxyde de sodium, puis on rince le tissu à l'eau, puis on le plonge dans une solution neutralisante.

11. Procédé selon l'une des revendications 1 à 10, **caractérisé en ce que** lors de l'étape de traitement à l'éthanol, on plonge une première fois le tissu dans une solution d'éthanol dont le titre en volume est supérieur à 90 %, et on plonge ensuite une deuxième fois le tissu dans une solution d'éthanol dont le titre en volume est supérieur à 95 %.

12. Procédé selon l'une des revendications 1 à 10, **caractérisé en ce que** l'on plonge une première fois le tissu dans une solution d'éthanol dont le titre en volume est de l'ordre de 95 %, et on plonge ensuite une deuxième fois le tissu dans une solution d'éthanol dont le titre en volume est de l'ordre de 100 %.

13. Utilisation d'un procédé selon l'une des revendications 1 à 12, dans le but de lutter contre les virus de la famille des Togaviridae, et de prévenir leur transmission lors des greffes de tissus.

14. Utilisation d'un procédé selon l'une des revendications 1 à 12, dans le but de lutter contre les virus du genre Alphavirus, et de prévenir leur transmission lors des greffes de tissus.

15. Utilisation d'un procédé selon l'une des revendications 1 à 12, dans le but de lutter contre les virus de la famille des Picormaviridae, et de prévenir leur transmission lors des greffes de tissus.

16. Utilisation d'un procédé selon l'une des revendications 1 à 12, dans le but de lutter contre les virus du genre Enterovirus, -notamment le virus Polio Sabin-, et de prévenir leur transmission lors des greffes de tissus

17. Utilisation d'un procédé selon l'une des revendications 1 à 12, dans le but de lutter contre les virus de la famille des Herpesviridae, et de prévenir leur transmission lors des greffes de tissus.

18. Utilisation d'un procédé selon l'une des revendications 1 à 12, dans le but de lutter contre les virus de la famille des Retroviridae, et de prévenir leur transmission lors des greffes de tissus.

19. Utilisation d'un procédé selon l'une des revendications 1 à 12, dans le but de lutter contre les virus du genre Lentivirus -notamment les virus de l'Immunodéficience humaine VIH-, et de prévenir leur transmission lors des greffes de tissus.

## Patentansprüche

1. Verfahren zur prophylaktischen Antivirusbehandlung von biologischen Geweben mit Kollagentextur für die Verwendung als Biomaterial, **dadurch gekennzeichnet, dass** nacheinander die folgenden Schritte durchgeführt werden:
- eine Behandlungsstufe mit Kohlendioxid CO₂ im hyperkritischen Zustand, im Verlaufe derer das Gewebe mit Kollagentextur mit einem Kohlendioxidstrom CO₂ im hyperkritischen Zustand in Kontakt gebracht wird, der die Aufgabe hat, Viruskontaminationen aus dem Inneren des Gewebes zu entfernen,
- eine Behandlungsstufe mit Wasserstoffperoxid H₂O₂, im Verlaufe derer das Gewebe mit Kollagentextur mit einer Wasserstoffperoxidlösung H₂O₂ in Kontakt gebracht wird, die die Aufgabe hat, Viruskontaminationen aus dem Gewebe zu entfernen,
- eine Behandlungsstufe mit Natriumhydroxid NaOH , im Verlaufe derer das Gewebe mit Kollagentextur mit einer Natriumhydroxidlösung NaOH in Kontakt gebracht wird, die die Aufgabe hat, Viruskontaminationen aus dem Gewebe zu entfernen,
- eine Behandlungsstufe mit Ethanol, im Verlaufe derer das Gewebe mit Kollagentextur mit einer Ethanollösung CH₃CH₂OH in Kontakt gebracht wird, die die Aufgabe hat, Viruskontaminationen aus dem Gewebe zu entfernen,
wobei diese Stufen so ausgelegt sind, dass ihre Aufeinanderfolge die Entfernung sämtlicher Viruskontaminationen aus dem behandelten Gewebe gewährleistet.

2. Verfahren nach Anspruch 1, **dadurch gekennzeichnet, dass** ein Kohlendioxidstrom CO₂ im hyperkritischen Zustand mit dem Gewebe mit Kollagentextur in einem Verhältnis von 100 bis 500 Gramm hyperkritischem Kohlendioxid pro Gramm Gewebe in Kontakt gebracht wird.

3. Verfahren nach einem der Ansprüche 1 und 2, **dadurch gekennzeichnet, dass** der genannte Kohlendioxidstrom CO₂ im hyperkritischen Zustand für eine Dauer mit dem Gewebe mit Kollagentextur in Kontakt gebracht wird, die in Abhängigkeit von der Leistung des genannten Stroms so bestimmt wird, dass 100 bis 500 Gramm hyperkritisches Kohlendioxid pro Gramm Gewebe durchfließen können.

4. Verfahren nach einem der Ansprüche 1 bis 3, **dadurch gekennzeichnet, dass** das hyperkritische Kohlendioxid CO₂ auf einem Druck zwischen 1 x 10⁷ und 5 x 10⁷ Pa und auf eine Temperatur zwischen 40°C und 55°C gebracht wird.

5. Verfahren nach einem der Ansprüche 1 bis 4, **dadurch gekennzeichnet, dass** das Gewebe mit Kollagentextur mit einer Wasserstoffperoxidlösung H₂O₂ in Kontakt gebracht wird, deren Massegehalt größer als 1% ist.

6. Verfahren nach einem der Ansprüche 1 bis 5, **dadurch gekennzeichnet, dass** das Gewebe mit Kollagentextur in Kontakt mit einer Wasserstoffperoxidlösung H₂O₂ gebracht wird, deren Massegehalt etwa 35% beträgt.

7. Verfahren nach einem der Ansprüche 1 bis 6, **dadurch gekennzeichnet, dass** das Gewebe mit Kollagentextur mit einer Wasserstoffperoxidlösung bei einer Temperatur zwischen 30°C und 50°C in Kontakt gebracht wird.

8. Verfahren nach einem der Ansprüche 1 bis 7, **dadurch gekennzeichnet, dass** das Gewebe mit Kollagentextur mit einer Natriumhydroxidlösung NaOH in Kontakt gebracht wird, deren Massegehalt zwischen 3% und 15% liegt.

9. Verfahren nach einem der Ansprüche 1 bis 7, **dadurch gekennzeichnet, dass** das Gewebe mit Kollagentextur mit einer Natriumhydroxidlösung NaOH in Kontakt gebracht wird, deren Massegehalt etwa 4% beträgt.

10. Verfahren nach einem der Ansprüche 1 bis 9, **dadurch gekennzeichnet, dass** während des Behandlungsschrittes mit Natriumhydroxid das Gewebe mit Kollagentextur mit einer Natriumhydroxidlösung in Kontakt gebracht, dann mit Wasser abgespült und anschließend in eine neutralisierende Lösung getaucht wird.

11. Verfahren nach einem der Ansprüche 1 bis 10, **dadurch gekennzeichnet, dass** während des Behandlungsschrittes mit Ethanol das Gewebe ein erstes Mal in eine Ethanollösung getaucht wird, deren Volumengehalt größer als 90% ist, und das Gewebe dann ein zweites Mal in eine Ethanollösung getaucht wird, deren Volumengehalt größer als 95% ist.

12. Verfahren nach einem der Ansprüche 1 bis 10, **dadurch gekennzeichnet, dass** das Gewebe ein erstes Mal in eine Ethanollösung getaucht wird, deren Volumengehalt etwa 95% beträgt, und das Gewebe dann ein zweites Mal in eine Ethanollösung getaucht wird, deren Volumengehalt etwa 100% beträgt.

13. Anwendung eines Verfahrens nach einem der Ansprüche 1 bis 12 mit dem Ziel, Viren der Togaviridae-Familie zu bekämpfen und deren Übertragung bei Gewebetransplantationen zu verhüten.

14. Anwendung eines Verfahrens nach einem der Ansprüche 1 bis 12 mit dem Ziel, Viren der Gattung Alphavirus zu bekämpfen und deren Übertragung bei Gewebeübertragungen zu verhüten.

15. Anwendung eines Verfahrens nach einem der Ansprüche 1 bis 12 mit dem Ziel, Viren der Picormaviridae-Familie zu bekämpfen und deren Übertragung bei Gewebetransplantationen zu verhüten.

16. Anwendung eines Verfahrens nach einem der Ansprüche 1 bis 12 mit dem Ziel, Viren der Gattung Enterovirus-insbesondere den Virus Polio Sabin - zu bekämpfen und deren Übertragung bei Gewebetransplantationen zu verhüten.

17. Anwendung eines Verfahrens nach einem der Ansprüche 1 bis 12 mit dem Ziel, Viren der Herpesviridae-Familie zu bekämpfen und deren Übertragung bei Gewebetransplantationen zu verhüten.

18. Anwendung eines Verfahrens nach einem der Ansprüche 1 bis 12 mit dem Ziel, Viren der Retroviridae-Familie zu bekämpfen und deren Übertragung bei Gewebetransplantationen zu verhüten.

19. Anwendung eines Verfahrens nach einem der Ansprüche 1 bis 12 mit dem Ziel, Viren der Gattung Lentivirus-insbesondere menschliche Immundefizienzviren HIV - zu bekämpfen und deren Übertragung bei Gewebetransplantationen zu verhüten.

## Claims

1. Process for treating, for the purpose of an anti-viral prophylaxis, a collagen-based tissue intended to serve as a biomaterial, **characterized in that** the following are successively carried out:
- a step of treating with supercritical carbon dioxide CO₂, during which the collagen-based tissue is placed in contact with a stream of supercritical carbon dioxide CO₂ which is adapted to have a function of removing the viral contamination within the tissue,
- a step of treating with hydrogen peroxide H₂O₂, during which the collagen-based tissue is placed in contact with a hydrogen peroxide H₂O₂ solution which is adapted to have a function of removing the viral contamination of the tissue,
- a step of treating with sodium hydroxide NaOH, during which the collagen-based tissue is placed in contact with a sodium hydroxide NaOH solution which is adapted to have a function of removing the viral contamination of the tissue,
- a step of treating with ethanol, during which the collagen-based tissue is placed in contact with an ethanol CH₃CH₂OH solution which is adapted to have a function of removing the viral contamination of the tissue, these steps being adapted such that their sequence ensures the removal of all viral contamination in the treated tissue.

2. Process according to Claim 1, **characterized in that** a stream of supercritical carbon dioxide CO₂ is used in contact with the collagen-based tissue, at a rate of 100 to 500 grams of supercritical carbon dioxide per gram of tissue.

3. Process according to either of Claims 1 and 2, **characterized in that** the said stream of supercritical carbon dioxide CO₂ is left in contact with the collagen-based tissue for a given period which depends on the flow rate of the said stream, to allow the passage of 100 to 500 grams of supercritical carbon dioxide per gram of tissue.

4. Process according to one of Claims 1 to 3, **characterized in that** the supercritical carbon dioxide CO₂ is placed at a pressure of between 1 × 10⁷ and 5 × 10⁷ Pa and at a temperature of between 40°C and 55°C.

5. Process according to one of Claims 1 to 4, **characterized in that** the collagen-based tissue is placed in contact with a hydrogen peroxide H₂O₂ solution whose titre by mass is greater than 1%.

6. Process according to one of Claims 1 to 5, **characterized in that** the collagen-based tissue is placed in contact with a hydrogen peroxide H₂O₂ solution whose titre by mass is of the order of 35%.

7. Process according to one of Claims 1 to 6, **characterized in that** the collagen-based tissue is placed in contact with a hydrogen peroxide solution at a temperature of between 30°C and 50°C.

8. Process according to one of Claims 1 to 7, **characterized in that** the collagen-based tissue is placed in contact with a sodium hydroxide NaOH solution whose titre by mass is between 3% and 15%.

9. Process according to one of Claims 1 to 7, **characterized in that** the collagen-based tissue is placed in contact with a sodium hydroxide NaOH solution whose titre by mass is of the order of 4%.

10. Process according to one of Claims 1 to 9, **characterized in that**, during the step of treating with sodium hydroxide, the collagen-based tissue is placed in contact with a sodium hydroxide solution and the tissue is then rinsed with water, after which it is immersed in a neutralizing solution.

11. Process according to one of Claims 1 to 10, **characterized in that**, during the step of treating with ethanol, the tissue is immersed a first time in an ethanol solution whose titre by volume is greater than 90%, and the tissue is then immersed a second time in an ethanol solution whose titre by volume is greater than 95%.

12. Process according to one of Claims 1 to 10, **characterized in that** the tissue is immersed a first time in an ethanol solution whose titre by volume is of the order of 95%, and the tissue is then immersed a second time in an ethanol solution whose titre by volume is of the order of 100%.

13. Use of a process according to one of Claims 1 to 12, with the aim of combating viruses of the Togaviridae family, and of preventing their transmission during tissue grafts.

14. Use of a process according to one of Claims 1 to 12, with the aim of combating viruses of the Alphavirus genus, and of preventing their transmission during tissue grafts.

15. Use of a process according to one of Claims 1 to 12, with the aim of combating viruses of the Picormaviridae family, and of preventing their transmission during tissue grafts.

16. Use of a process according to one of Claims 1 to 12, with the aim of combating viruses of the Enterovirus genus, especially polio Sabin virus, and of preventing their transmission during tissue grafts.

17. Use of a process according to one of Claims 1 to 12, with the aim of combating viruses of the Herpesviridae family, and of preventing their transmission during tissue grafts.

18. Use of a process according to one of Claims 1 to 12, with the aim of combating viruses of the Retroviridae family, and of preventing their transmission during tissue grafts.

19. Use of a process according to one of Claims 1 to 12, with the aim of combating viruses of the Lentivirus genus,
